# EUROPEAN PATENT SPECIFICATION

(11) **EP 4 176 264 B1**
(45) Date of publication and mention of the grant of the patent: **03.09.2025**
(21) Application number: 21739679.5
(22) Date of filing: 06.07.2021
(51) Int. Cl.: G01N 33/569, A61K 39/00, C07K 14/00, G01N 33/68

(54) **CD1 PEPTIDE-EPITOPE FOR USE IN THE TREATMENT OF A DISEASE CAUSED BY AN INTRACELLULAR PATHOGEN**
CD1 PEPTIDE-EPITOP ZUR VERWENDUNG BEI DER BEHANDLUNG EINER KRANKHEIT, DIE DURCH EIN INTRAZELLULÄRES PATHOGEN VERURSACHT WIRD
UTILISATION D'UN EPITOPE-PEPTIDE DE CD1 DANS LE TRAITEMENT D'UNE MALADIE CAUSÉE PAR UN PATHOGÈNE INTRACELLULAIRE

(30) Priority: 06.07.2020 EP 20184175
(43) Date of publication of application: 10.05.2023
(73) Proprietor: Equaly S.A., 1348 Louvain-la-Neuve (BE)
(72) Inventor: SAINT-REMY, Jean-Marie, 1390 Grez-Doiceau (BE)
(74) Representative: Kirkpatrick
(86) International application number: PCT/EP2021/068585
(87) International publication number: WO 2022/008479

(56) References cited:
- EP-A1- 3 388 447

## Description

### FIELD OF THE INVENTION

The present invention is in the field of immunology for the specific treatment of intracellular pathogens upon a strong cytotoxic response dominated by cytotoxic T cells.

### BACKGROUND OF THE INVENTION

Intracellular pathogens such as viruses or bacteria represent a major concern as no real robust specific solution does exist.

Indeed, the response towards infections with viruses requires activation of a strong cytotoxicity component dominated by T cells of the CD8 lineage and natural killer (NK) cells. This is obtained naturally by recognition of membrane-bound and cytosolic-distributed pathogen-associated molecular patterns (PAMPS) by a number of pattern-recognition receptors (PPRs) of the host, including toll-like receptors (TLRs), RIG-like receptors (RIGs) and Nod-like receptors (NLRs). This is followed by activation of various signaling pathways involving NF-kB and IRFs ending into the production of cytokines, in particular type I and type III interferons.

However, virtually each single step in these activation pathways can be the target of virus interference or proteolysis, with, as a result, a weakening of innate and cytotoxic immune responses. Should the pathogen escape to the immune system immediately after the infection, it becomes resistant, either invisible to the immune system, or able to modulate the host-cell to escape the immune system.

Chemotherapies, including potent antibiotics and antiviral molecules, represent a solution, but associated to significant side-effects and often unable to kill all the pathogens hidden into host cells or in tissular niches acting as reservoirs, meaning that, when the treatment has to be stopped, for instance to avoid major problems to the patient due to these side-effects, the pathogen can again grow and infect other cells or tissues of the patient.

Even preventive or curative (therapeutic) vaccination is not potent enough to elicit the correct immune response towards all the intracellular pathogens: sometimes it has been possible to develop a vaccine, sometimes all the previous attempts have failed.

Vaccination strategies classically make use of virus- or pathogen-derived proteins, or of their encoding sequences in the format of DNA or RNA, administered together with an adjuvant. Such vaccination preferentially boosts the production of specific antibodies in a context of CD4+ T cells recruitment, yet develops little if any cytotoxicity.

In addition to this inherent limitation, the strength of the response to a vaccine is dependent on the ability of MHC class II determinants to present virus or pathogen epitopes to CD4+ T cells. The large polymorphism of MHC class II determinants within the population makes the efficacy of CD4+ T cell activation very variable between individuals, adding another uncertainty in the prediction of vaccine efficacy and induction of memory. Indeed, it is common observation to see an antibody response towards viruses disappearing with time.

There is therefore an urgent need to develop vaccination strategies which would circumvent as much as feasible the preferential recruitment of CD4+ T cell to the detriment of cytotoxic cells, as well as to cope with inherent variations in responses due to class II MHC polymorphism.

On the other hand, the capacity of the CD1 molecules to load peptide epitopes, especially those bearing hydrophobic motives at specific locations, has been identified by the inventor of the present patent application and developed into therapeutic applications, for instance in the patent applications WO 2012/069572, WO 2012/069575, WO 2013/174805 and WO 2018/189405 (EP3388447).

EP3388447 A1 describes the use of CD1 peptide-epitopes in the prevention or treatment of diseases caused by an intracellular pathogen.

However, a CD1-based direct approach is still not widely recognized as a tool in immunology. In fact, the main purpose of these patent documents is to modify such peptide-epitopes to specifically modulate the immune response, not to directly use them in their native form in vaccination purposes and/or to train the immune system.

### SUMMARY OF THE INVENTION

The present invention relates to a CD1 peptide-epitope for use in the prevention or in the treatment of a disease caused by an intracellular pathogen, wherein:
(i) the said disease is preferably a viral disease caused by a virus preferably selected from the group consisting of *Flaviviridae, Coronaviridae, Orthomyxoviridae, Herpesviridae and Picornaviridae,*
(ii) the said CD1 peptide-epitope is a fragment of a protein expressed by the said intracellular pathogen, wherein the said CD1 peptide-epitope comprises the sequence X₁X₂X₃X₄X₅X₆X₇, wherein the said X₂, X₃, X₄, X₅, and X₆ independently stand for any amino acid, and wherein the said X₁ and X₇ residues are independently {F;W;T;H or Y}, and
(iii) said use comprises administration of the said intracellular pathogen in an inactivated form or administration of a second peptide based on a protein of the said intracellular pathogen, wherein administration of said inactivated intracellular pathogen or said second peptide is performed subsequent to administration of the CD1 peptide-epitope and wherein said second peptide comprises the CD1 epitope sequence defined in (ii) and at least one MHC Class I epitope.

Furthermore, the present disclosure describes, but does not claim, a (an *in vitro*) method to identify one or several peptide-epitope(s) to activate NKT cells in a Th1-like (IFNγ and/or IL-12) and/or cytotoxic response towards an intracellular pathogen comprising the steps of identifying a CD1 peptide epitope in the said intracellular pathogen, and of measuring *(in vitro)* the capacity of the identified CD1 peptide epitope to activate NKT cells in a Th1-like and/or cytotoxic response.

Preferably, in this *(in vitro)* method, the NKT cell activation is measured upon the incubation of the peptide-epitope with cells expressing CD1 molecule at their surface, followed by the addition of a population of NKT cells and the determination of activation of said NKT cells.

Preferably, in this *(in vitro)* method, the identification of the CD1 peptide epitope is measured upon the capacity of the peptide (or of a fragment of a peptide) of the intracellular pathogen to bind to this CD1 molecule.

Preferably, in this *(in vitro)* method, the identified CD1 peptide epitope has the sequence X₁X₂X₃X₄X₅X₆X₇, wherein these X₂X₃X₄X₅X₆ independently stand for any amino acid, wherein these X₁ and X₇ residues are independently {F;W;T;H or Y}, wherein, preferably X₄ is {I;V;L or M} and/or wherein, preferably, at least one X₁ or X₇ residue is {F;W or Y} and/or wherein, preferably this X₁X₂X₃X₄X₅X₆X₇ sequence belongs to and/or forms an alpha-helix, possibly after binding to CD1.

Advantageously, in this *(in vitro)* method, the identified CD1 peptide epitope does not comprise an MHC class II epitope.

Preferably, in this *(in vitro)* method, the activation of NKT cells is determined by the quantification of interferon (IFN)-y and/or Interleukin (IL)-12 levels, possibly IL-4, IFNα and/or IFNβ is (are) also quantified and the IFNy/IL-4 (or IL-12/IL-4) ratio is measured; an increase meaning a cytotoxic response.

A related not claimed aspect of the present disclosure is a CD1 peptide-epitope for use in the prevention or in the treatment of a disease, preferably a disease caused by an intracellular pathogen, wherein this disease is preferably a viral disease being more preferably selected from the group consisting of *Flaviviridae, Coronaviridae, Orthomyxoviridae, Herpesviridae and Picornaviridae* and wherein this CD1 peptide epitope is a fragment of a protein expressed by this intracellular pathogen, this fragment, advantageously, not comprising a MHC Class II epitope.

Advantageously, this CD1 peptide-epitope (for use in the prevention or in the treatment of a disease caused by an intracellular pathogen) is obtainable by the above-described *(in vitro)* method.

Another related aspect of the present invention is a kit of parts (for use in the treatment of a disease, preferably a disease caused by an intracellular pathogen) comprising a first peptide being the CD1 peptide epitope described here above (advantageously not comprising an MHC Class II epitope) and a second peptide derived from this intracellular pathogen and being a peptide comprising this first peptide epitope in addition to other epitopes, advantageously at least one MHC Class II epitope.

This kit of parts is preferably for use in the treatment of a viral disease, more preferably selected from the group consisting of *Flaviviridae, Coronaviridae, Orthomyxoviridae, Herpesviridae and Picornaviridae.*

Preferably, this kit of parts further comprises an adjuvant for the vaccination with the first peptide (CD1 peptide epitope, advantageously not comprising an MHC Class II epitope) and/or an adjuvant for the vaccination with the second peptide (advantageously comprising the CD1 peptide epitope and MHC Class II epitope, possibly also other MHC Class I or II epitopes).

Preferably, this kit of parts further comprises means to quantify the IgG1 and/or IgG3 specific for the CD1 peptide epitope (or total IgG1 and/or IgG3) and/or means to quantify all the IgGs (or at least IgG2), or the IgGs other than IgG1 and/or IgG3 specific for the CD1 peptide epitope (or total IgG or of the IgG other than IgG1 and/or IgG3, such as IgG2).

Preferably, this kit of parts further comprises means to quantify the IgG1 and/or IgG3 specific for the second peptide (or total IgG1 and/or IgG3) and/or means to quantify all the IgGs (or IgG2) specific for the second peptide or all the IgGs (IgG2) other than these IgG1 and/or IgG3 specific for the second peptide (or total IgG, or of the IgGs other than IgG1 and/or IgG3, such as IgG2), these IgGs (IgG2), other than IgG1 and IgG3 possibly being nonspecific for the (first) CD1 peptide epitope.

Advantageously, the ratio {IgG1 and/or IgG3}:IgGs (or IgG2) is measured, and/or an (a relative) increase of (specific) IgG1 and/or of IgG3 points to a cytotoxic response of the immune system, which reflects a correct response to the treatment.

Preferably, this kit of parts further comprises means to quantify IFNγ and/or IL-12 and/or IL-4 and/or IFNα and/or IFNβ Levels.

Another related, but not claimed, aspect of the present disclosure is a pharmaceutical method to condition the immune system of a patient towards a specific Th1-like and/or cytotoxic response against an intracellular pathogen comprising the steps of identifying a CD1 peptide epitope in a protein expressed by the said intracellular pathogen, and of measuring *(in vitro)* the capacity of the identified CD1 peptide epitope to activate NKT cells in a Th1-like (IFNγ and/or IL-12) response and/or to elicit a cytotoxic response, and of administering a first peptide, being (essentially) this CD1 peptide epitope, to a patient, preferably together with a vaccine adjuvant.

Preferably, in this method, the step of identifying a CD1 peptide epitope in the intracellular pathogen comprises the determination of the capacity of the peptide to bind to the said CD1 molecule.

Preferably, this method further comprises the incubation of the peptide-epitope with cells carrying CD1, followed by addition of a population of NKT cells and the determination of activation of these NKT cells.

Preferably, in this method, the (cytotoxic) activation of NKT cells is determined by the quantification of IFNγ and/or IL-12 levels (also advantageously of IFNα and/or IFNβ levels, and possibly of IL-4).

Preferably, in this method, the epitope has the consensus sequence X₁X₂X₃X₄X₅X₆X₇, from which these X₂X₃X₄X₅X₆ independently stand for any amino acid, from which these X₁ and X₇ residues are independently {F;W;T;H or Y}, wherein, preferably X₄ is {I;V;L or M} and/or wherein, preferably, at least one X₁ or X₇ residue is {F;W or Y} and/or wherein, preferably the said X₁X₂X₃X₄X₅X₆X₇ sequence belongs to and/or forms an alpha-helix, possibly after binding to CD1.

Preferably, this method further comprises the step of measuring (ex *vivo*) a specific cytotoxic response towards the said epitope from a sample from the patient after the administration of the peptide epitope.

Advantageously, in this pharmaceutical method, the peptide comprising the CD1 epitope does not comprise a Class II MHC epitope.

Preferably, this, not claimed, pharmaceutical method, further comprises a subsequent step of administering to the patient a vaccine being a second peptide comprising a Class II MHC epitope, preferably together with an adjuvant and being preferably a peptide further comprising the CD1 peptide epitope, and possibly comprising a Class I or Class II MHC epitope.

Preferably, this method comprises the step of measuring the level of IgG1 and/or of IgG3 (from a blood sample from the patient), advantageously able to recognize the said CD1 peptide-epitope, possibly after the injection of the CD1 peptide-epitope (not comprising a Class II MHC epitope) and/or after the injection of the second peptide (comprising the CD1 peptide-epitope and the Class II MHC epitope).

In a further aspect, the present invention relates to a nucleotide sequence encoding a CD1 peptide-epitope for use in the prevention or in the treatment of a disease caused by an intracellular pathogen, wherein
(i) the said disease is preferably a viral disease caused by a virus preferably selected from the group consisting of *Flaviviridae, Coronaviridae, Orthomyxoviridae, Herpesviridae and Picornaviridae,*
(ii) the said CD1 peptide-epitope is a fragment of a protein expressed by the said intracellular pathogen, wherein the said CD1 peptide-epitope comprises the sequence X₁X₂X₃X₄X₅X₆X₇, wherein the said X₂, X₃, X₄, X₅, and X₆ independently stand for any amino acid, and wherein the said X₁ and X₇ residues are independently {F;W;T;H or Y}, and
(iii) said use comprises administration of the said intracellular pathogen in an inactivated form or administration of a second peptide based on a protein of the said intracellular pathogen, wherein administration of said inactivated intracellular pathogen or said second peptide is performed subsequent to administration of the nucleotide sequence encoding the CD1 peptide-epitope and wherein said second peptide comprises the CD1 epitope sequence defined in (ii) and at least one MHC Class I epitope.

### DETAILED DESCRIPTION OF THE INVENTION

The inventor has found that the vaccination of patients against intracellular pathogens including viruses, as classically done in the art, is sometimes inefficient.

On the other hand, the inventor has found that, when a vaccination procedure is preceded by administration of a CD1-restricted epitope (or of the encoding nucleotide sequences) of the same virus or intracellular pathogen, an immune response towards a virus or an intracellular pathogen is forced into enhanced cytotoxicity including CD8+ T cells, NKT and NK cells, together with significantly increased concentrations of specific antibodies and activation of CD4+ T cells, as compared to the vaccination by (classical) administration of a peptide of the virus or pathogen together with an adjuvant. In other words, although chronic infection mostly results from strategies developed by pathogens to subvert an adaptive immunity, the inventor has found that forcing an innate response provides a boost towards an efficient adaptive response.

As a pioneer in having identified the possibility to modulate the immune system, based on CD1-peptide interaction (in the present invention, unless otherwise stated, the term "CD1" means collectively CD1a, CD1b, CD1c and CD1d, but not CD1e), the inventor has found that the vaccination of a patient with CD1 epitopes elicits a, or promotes a subsequent, local and/or specific Th1-like response with cytotoxic functions and/or the local and/or specific production of IFNγ and/or of IL-12 cytokines. Another hallmark of the NKT activation to trigger a cytotoxic response is their expression level of perforin and granzymes.

As such, this response represents a significant added value over the classic vaccination, since peptides selected on a different, more focused, basis elicit a more predictable response of the immune system, in the present case a focused Th1-like response, rather than a less-predictable response.

Indeed, the present invention allows to elicit a strong immune response dominated by cytotoxic T cells in a patient while avoiding the deleterious effects of (i) exogenous molecules modulating the immunity in a non-selective way, as well as of (ii) an improper response of the patient's body towards the intracellular pathogen Moreover, the inventor has found that such CD1-focused vaccination synergizes with a subsequent more classical vaccination scheme not necessarily focused on CD1/NKT and cytotoxic response, because the immune system has been conditioned for the correct response first: the response to the subsequent vaccination is now correctly oriented, and boosted.

Therefore, a first aspect of the present invention is a CD1 peptide-epitope for use in the prevention or in the treatment of a disease caused by an intracellular pathogen, wherein
(i) the said disease is preferably a viral disease caused by a virus preferably selected from the group consisting of *Flaviviridae, Coronaviridae, Orthomyxoviridae, Herpesviridae and Picornaviridae,*
(ii) the said CD1 peptide-epitope is a fragment of a protein expressed by the said intracellular pathogen, wherein the said CD1 peptide-epitope comprises the sequence X₁X₂X₃X₄X₅X₆X₇, wherein the said X₂, X₃, X₄, X₅, and X₆ independently stand for any amino acid, and wherein the said X₁ and X₇ residues are independently {F;W;T;H or Y}, and
(iii) said use comprises administration of the said intracellular pathogen in an inactivated form or administration of a second peptide based on a protein of the said intracellular pathogen, wherein administration of said inactivated intracellular pathogen or said second peptide is performed subsequent to administration of the CD1 peptide-epitope and wherein said second peptide comprises the CD1 epitope sequence defined in (ii) and at least one MHC Class I epitope.

In one embodiment, X₄ is {I;V;L or M}. In another embodiment, at least one X₁ or X₇ residue is {F;W or Y}. In a further embodiment, the said X₁X₂X₃X₄X₅X₆X₇ sequence belongs to and/or forms or has the capacity to form an alpha-helix.

In one embodiment, the CD1 peptide-epitope, i.e. the peptide used for the first administration, does not comprise an MHC class I epitope.

In one embodiment, the CD1 peptide-epitope, i.e. the peptide used for the first administration, does not comprise an MHC class II epitope.

In one embodiment, said second peptide, i.e. the peptide used for the second administration, further comprises at least one MHC Class II epitope.

A further related, but not claimed, aspect of the present disclosure is a pharmaceutical method to condition the immune system of a patient towards a specific Th1-like and/or cytotoxic response against an intracellular pathogen comprising the steps of identifying (*in silico*) a (putative) CD1 (CDla, CDlb, CD1c and/or CD1d) peptide epitope in the said intracellular pathogen, of measuring *in vitro* the capacity of the identified (putative) CD1 (CDla, CDlb, CD1c and/or CD1d) peptide epitope to activate NKT cells in a cytotoxic response (IFNγ and/or IL-12 production; perforin and granzymes expression; also the measurement of 3H thymidine incorporation and/or of NUR77 expression) and of administering (*i.e.* first vaccination step, first vaccine; see below for the second vaccine) the said (validated) CD1 (CDla, CDlb, CD1c and/or CD1d) peptide epitope to a patient, preferably together with an adjuvant. Possibly, instead of (directly) administering the CD1 peptide epitope, the nucleotide sequence encoding such CD1 peptide epitope is administered to the patient.

The preferred pathogens are viruses, such as DNA viruses, RNA viruses such as *flaviviridae* (West Nile virus, Dengue virus) or *Coronaviridae,* but also bacteria and mycobacteria, such as *Mycobacterium tuberculosis* and other mycobacteria pathogenic for humans or animals, *Yersinia, Brucella, Chlamydiae,* mycoplasma, Rickettsiae, as well as protozoan parasites, for instance *Leishamania sp.,* Trypanosoma *sp.,* Toxoplasma *sp.,* Listeria *sp.* and Histoplasma sp.

The *in silico* identification is preferably achieved upon firstly the identification of the intracellular pathogen, then, optionally, the screen of suitable proteins, then the screen of motives susceptible to be bound by CD1 molecules (CDla, CDlb, CD1c and/or CD1d). The capacity of the peptide (comprising the (putative) epitope) to bind to the CD1 (CDla, CDlb, CD1c and/or CD1d) molecule is advantageously further determined *in vitro.*

Preferably, in this method, the NKT cell activation is measured upon the incubation of the identified (putative) peptide-epitope (*in silico* and/or after the above *in vitro* binding step) with cells carrying CD1 (CDla, CDlb, CD1c and/or CD1d), followed by the addition of a population of NKT cells and the determination of activation of these NKT cells, for instance by their capacity to exert a cytotoxic effect on reporter cells.

Advantageously, the activation of NKT cells is (also) determined by the quantification of IFNγ and/or IL-12 levels (quantification of the mRNA or of the protein level; see below) or of perforin and/or granzymes expression and/or NUR77 level(s), as well as the incorporation of 3H thymidine (by NKT cells). This allows to ensure that the selected peptide epitope will elicit the correct cytotoxic immune response, when injected to a patient.

In this (*in silico* step of this) method, preferably, the peptide epitope is a (CD1, such as a CD1d) peptide epitope having the consensus sequence X₁X₂X₃X₄X₅X₆X₇, wherein such X₂X₃X₄X₅X₆ independently stand for any amino acid, wherein the X₁ and X₇ residues are independently {F;W;T;H or Y}, wherein, preferably X4 is {I;V;L or M} and/or wherein, preferably, at least one X₁ or X₇ residue is {F;W or Y} and/or wherein, preferably, the X₁X₂X₃X₄X₅X₆X₇ sequence forms a, and/or is part of an alpha helix, possibly after binding to CD1. The presence of at least one F, W or Y residue on X₁ or on X₇ ensures a strong anchoring on CD1.

The configuration in helix is also beneficial for a good affinity to CD1. This implies that the other amino acids are compatible with an alpha helix, especially the amino acid at position X₄.

The presence of an alpha helix can be predicted *in silico* using readily available algorithms. In the present invention, several amino acids, such as proline or glycine, which are known to break alpha helices, are nevertheless acceptable at certain positions. For instance, a glycine at residues X₂, X₃, X₅, or X₆ is possible, since such residue allows for more flexibility, which is beneficial for CD1 fixation. Conversely, a proline residue (e.g. at a putative X₀ or X_{-1 -2 -3} position) can be the first amino acid of an alpha helix. On the other hand, bulky amino acids, such as arginine or tryptophan, are not preferred at intermediate positions X₂-X₆, whereas tryptophan can be present, and even is one of the preferred amino acid at X₁ and/or X₇.

Besides the *in silico* screening, the potential for forming an alpha helix can be tested by chemical analysis, such as NMR or X-Ray, preferably after loading to CD1.

Thus a preferred CD1 sequence has a W, F or Y residue on X₁ and/or X₇ and forms (or has the capacity to form) an alpha helix.

Also a preferred CD1 sequence has a W, F or Y residue on X₁ and/or X₇ and a I, L, M or V residue on X₄.

The most preferred CD1 sequences have a W, F or Y residue on X₁ and/or X₇ and a I, L, M or V residue on X₄ and form (or have the capacity to form) an alpha helix.

In this method, alternatively or in addition, the peptide epitope (forming or having the capacity to form an alpha helix) is selected by a first *in silico* screening step based on the identification of clustering of hydrophobic amino acids within a peptide (e.g. at least 2 hydrophobic amino acids over a stretch of up to 12 amino acids, up to 10, up to 7 amino acids, preferably, at least 3 hydrophobic amino acids over a stretch of up to 12 amino acids, up to 10, up to 7 amino acids, or at least 4 amino acids over a stretch of up to 12 amino acids, up to 10, up to 7 amino acids.

The wording "hydrophobic amino acids" is well-understood by one skilled in the art. In the context of the present invention, this preferably refers to methionine (M), leucine (L), isoleucine (I), phenylalanine (F), tyrosine (Y) and tryptophan (W).

In this *in silico* screening (including the X₁-X₇ *in silico* screening and the alternative *in silico* screening), preferably, the CD1 epitope does not comprise a Class II MHC epitope. Such epitope, although desirable in classical vaccination, including in the subsequent step of vaccination (see below), may reduce, potentially strongly reduce, the CD1-driven response of the immune system.

One convenient way to ensure this is to select peptides short enough and/or to select peptides where predicted Class II MHC epitopes are not present (e.g. truncated peptides where putative Class II MHC epitopes have been removed). Suitable peptides can be selected by methods known in the art. For example, Poluektov et al. Vaccine 39: 2110-2216, 2021 describes epitope selection for SARS-CoV2.

Indeed, one convenient way to avoid Class II MHC epitope is to design short peptides comprising these CD1 epitopes, for instance of less than 50, less than 40, less than 30, less than 20 amino acids. A minimal peptide comprising the CD1 epitope can have a size of 11 amino acids, including the 7-amino acid consensus depicted here above. Thus, in one embodiment, the CD1 peptide-epitope for use according to the invention is a fragment (i.e. a contiguous fragment) of a protein expressed by the intracellular pathogen of between 11 and 50 amino acids in length, such as between 11 and 40, or between 11 and 30, or between 11 and 20 amino acids in length. In another embodiment, the CD1 peptide-epitope for use according to the invention further contains flanking sequences that do not correspond to the flanking regions in the protein expressed by the intracellular pathogen. Thus, in such embodiments, the CD1 peptide-epitope comprises a fragment of a protein expressed by the intracellular pathogen of between 11 and 50 amino acids in length, such as between 11 and 40, or between 11 and 30, or between 11 and 20 amino acids in length.

Indeed, it is preferred that the CD1 epitope still forms, or has the capacity to form, an alpha helix, which implies some biophysical constraints, including with regards to the minimal size of the epitope and thus also the presence of flanking residues: such residues are not directly involved for the CD1 fixation, yet they impart a conformation to the whole sequence, which affects its capacity to bind to CD1.

Another way to exclude Class II MHC epitope, for instance if longer peptides are intended to be designed, is to rely on *in silico* analysis.

Another practical way is to rely on a cellular test where cells are transfected with Class II human MHC haplotypes (several cell lines, each transfected with a different Class II human MHC haplotype), then tested so as to check whether the peptide comprising the CD1 epitope also qualifies as a Class II MHC epitope, and to reject such peptides. For example, 15 to 20 of the predominant MHC haplotypes could be tested.

This method, preferably, further comprises the step of measuring ex *vivo* a specific cytotoxic response towards the CD1 epitope. This allows to ensure, beside a direct cytotoxic action of the NKT cells, a correct activation of the immune system before administration of the second peptide-epitope to the patient (see here below the second vaccination step).

In one embodiment of the CD1 peptide-epitope for use in a treatment as claimed, said treatment further comprises a subsequent second vaccination step of administering to the patient a vaccine, preferably based on the same protein comprising the CD1 epitope expressed by the intracellular pathogen, preferably together with an adjuvant. This vaccine comprises a second peptide which comprises at least one second epitope which is different from the CD1 peptide-epitope. Preferably this second vaccine comprises at least one MHC Class I epitope and at least one MHC Class II epitope. A preferred second vaccine advantageously comprises both the CD1 epitope of the first vaccine and this MHC Class II epitope. Suitable MHC Class II epitopes can be identified using the methods described above, i.e. in silico prediction methods and/or testing on cell lines expressing Class II human MHC haplotypes. Suitable MHC Class I epitopes can be identified by methods known in the art, for example those described in Yang et al. BMC Bioinformatics 22:231, 2021.

This second vaccination step of the treatment for which the use of the CD1 peptide-epitope is claimed may be performed after having ensured that the NKT cells have been correctly activated by the first (CD1-restricted) vaccine to elicit a cytotoxic response.

The epitope(s) or peptide(s) used for this second vaccination step is (are) not necessarily focused on CD1 epitopes: peptides bearing multiple epitopes as well as the peptides bearing MHC Class II epitopes can be used and, in fact, are advantageously used. The peptide(s) used in this subsequent vaccination can also comprise (putative) CD1 epitopes and even validated CD1 epitopes, preferably the CD1 epitope used in the first vaccination.

In some embodiments, the second step of the treatment for which the use of the CD1 peptide-epitope is claimed comprises administration of a full-length protein of the intracellular pathogen, preferably the protein which also comprises the CD1 peptide-epitope, or an inactivated form of the pathogen, such as a live-attenuated virus.

In one embodiment, the pathogen is Dengue virus and the CD1 peptide-epitope is a fragment of an NS protein, preferably NS5 and comprising or consisting of the sequence set forth in SEQ ID NO:1. Preferably, the CD1 peptide-epitope is between 11 and 50 amino acids in length, such as between 11 and 40, or between 11 and 30, or between 11 and 20 amino acids in length. In a further embodiment hereof, the second peptide is a full-length NS5 protein.

In another embodiment, the pathogen is Coxsackie B4 virus and the CD1 peptide-epitope is a fragment of the polyprotein, preferably comprising or consisting of the sequence set forth in SEQ ID NO:2. Preferably, the CD1 peptide-epitope is between 11 and 50 amino acids in length, such as between 11 and 40, or between 11 and 30, or between 11 and 20 amino acids in length. In a further embodiment hereof, the second peptide is a full-length polyprotein.

In another embodiment, the pathogen is SARS-CoV2 virus and the CD1 peptide-epitope is a fragment of the S protein, preferably comprising or consisting of the sequence set forth in SEQ ID NO:3. Preferably, the CD1 peptide-epitope is between 11 and 50 amino acids in length, such as between 11 and 40, or between 11 and 30, or between 11 and 20 amino acids in length. In a further embodiment hereof, the second peptide is a full-length S protein.

In another embodiment, the pathogen is influenza A virus and the CD1 peptide-epitope is a fragment of the hemagglutinin protein, preferably comprising or consisting of the sequence set forth in SEQ ID NO:4. Preferably, the CD1 peptide-epitope is between 11 and 50 amino acids in length, such as between 11 and 40, or between 11 and 30, or between 11 and 20 amino acids in length. In a further embodiment hereof, the second peptide is a full-length hemagglutinin.

In another embodiment, the pathogen is herpes simplex virus and the CD1 peptide-epitope is a fragment of the Vp16 protein, preferably comprising or consisting of the sequence set forth in SEQ ID NO:5. Preferably, the CD1 peptide-epitope is between 11 and 50 amino acids in length, such as between 11 and 40, or between 11 and 30, or between 11 and 20 amino acids in length. In a further embodiment hereof, the vaccination is with a live-inactivated HSV vaccine.

A related, but not claimed, aspect of the present disclosure is an *in vitro* method to identify peptide-epitopes to activate NKT cells in eliciting a (subsequent) cytotoxic response (IFNγ and/or IL-12; perforin, granzymes and/or NUR77 expression level; 3H thymidine incorporation) towards an intracellular pathogen comprising the steps of identifying a CD1 (CDla, CDlb, CD1c and/or CD1d) epitope in this intracellular pathogen, and of measuring *in vitro* the capacity of the identified CD1 (CDla, CDlb, CD1c and/or CD1d) epitope to activate NKT cells in a cytotoxic response.

Preferably, in this *in vitro* method, the CD1 epitope (CDla, CDlb, CD1c and/or CDld epitope(s)) is (are) identified upon the capacity of a peptide to bind to this (these) CD1 (CDla, CDlb, CD1c and/or CD1d) molecule(s).

Preferably (or in addition to the binding to CD1), this peptide potentially binding to this (these) CD1 (CDla, CDlb, CDlc and/or CDld molecule(s)) is identified in a preliminary (*in silico*) step as depicted in the pharmaceutical method here above (presence of a stretch of hydrophobic amino acids or the presence of the X₁X₂X₃X₄X₅X₆X₇ epitope with specific amino acids at positions 1, 7 (F/W/T/H/Y) and possibly also at position 4 (I/L/M/V).

Preferably, or in addition, in this *in vitro* method, the NKT cell activation is measured upon the incubation of the peptide-epitope with cells carrying CD1 (CDla, CDlb, CD1c and/or CD1d), followed by addition of a population of NKT cells and by the determination of the activation of these NKT cells.

Preferably, in this method (also possibly for the above pharmaceutical method or for the pharmaceutical product here below), the activation of NKT cells is determined by the quantification of IFNγ and/or IL-12 levels, for instance based on the expression level of the mRNA for IFNγ and/or IL-12 or on the amount of the protein in the surrounding (conditioned) cell culture medium, for instance by ELISA measurement. Perforin and granzymes expression levels can also be advantageously measured, as well as NUR77 (mRNA level) or the incorporation of 3H thymidine (in such NKT cells).

A related aspect of the present invention is a CD1 (CDla, CDlb, CD1c and/or CD1d) peptide-epitope (vaccine) for use in the treatment of a disease caused by an intracellular pathogen as claimed, preferably a virus.

Alternatively, instead of a direct administration of the CD1 peptide epitope, the corresponding nucleotide sequence expressing this CD1 peptide epitope can be administered to the patient.

Although not really limited to a particular viral disease or virus, this viral disease is preferably selected from the group consisting of *Flaviviridae* (West Nile virus, Dengue virus), *Coronaviridae (SARS, such as SARS-CoV-2), Orthomyxoviridae (virus causing influenza), Herpesviridae (Herpes virus, including Epstein-Barr virus (EBV)) and Picornaviridae.* Viral diseases, in the context of the present invention, also include malignant diseases associated with viruses, for example lymphoma associated with EBV, in particular EBV-associated lymphomas occurring after an organ transplant, such as a liver transplant.

Also intracellular infections with bacteria and mycobacteria such as *Mycobacterium tuberculosis* and other mycobacteria pathogenic for humans or animals, *Yersinia, Brucella, Chlamydiae,* mycoplasma, Rickettsiae, as well as protozoan parasites, for instance *Leishamania sp.,* Trypanosoma *sp.,* Toxoplasma *sp.,* Listeria *sp.* and Histoplasma sp can be treated.

Preferably, this CD1 (CDla, CDlb, CD1c and/or CD1d) peptide-epitope is obtainable as in the above *in vitro* method and/or following the same approach as in the above *in vitro* method to identify peptide-epitopes to activate NKT cells, with the specific *in silico* step(s), *in vitro* (CD1 binding) step and/or the step for measuring *(in vitro)* the activation of NKT cells.

Another related, but not claimed, aspect of the present disclosure is a kit of parts for use in the treatment of a disease caused by an intracellular pathogen, comprising a CD1 (CDla, CDlb, CD1c and/or CD1d) peptide epitope (preferably the CD1a, CDlb, CD1c and/or CDld peptide epitope described here above and not comprising a Class II MHC epitope) and a second peptide, preferably comprising a Class II MHC epitope derived from the intracellular pathogen, preferably belonging to the same protein bearing this CD1 peptide epitope, more preferably the second peptide comprises at least the CD1 peptide epitope and a (one or several) Class II MHC epitope.

This kit of parts is advantageously for use in the treatment of a disease caused by an intracellular pathogen such as a virus, also an intracellular bacterium (see above).

Preferably, this kit of parts further comprises an adjuvant for the vaccination with the CD1 (CDla, CDlb, CD1c and/or CD1d) peptide epitope and/or an adjuvant for the vaccination with the second peptide.

The preferred adjuvants are selected for their capacity to elicit an inflammatory response, such as lipidic emulsions. Alum adjuvant is not preferred for the CD1 peptide epitope.

### Examples

### 1. Dengue virus infection

Dengue is a major public health issue in tropical and subtropical regions. This ssRNA enveloped Flavivirus is responsible for a fever syndrome that can adopt a severe hemorrhagic form leading to a shock syndrome. Though the virus is activating an interferon a/b response, the latter is severely impaired by a number of mechanisms preventing an efficient response. Eliciting a strong innate response towards dengue virus would help in controlling infection by its capacity to trigger a CD8+ T cell response known to be protective, whilst the precise role of antibodies is still debated.

Non-structural (NS) proteins are used to develop a vaccination strategy, considering that an immune response to NS is essential to generate efficient protection against the virus. Thus, NS5 is chosen for the following experiments, based on its importance in blocking signals required for production of interferons alpha and beta.

The sequence of NS5 shows a determinant with the characteristics required to bind to CD1, namely,
AG-HGQVDNF-SL, (SEQ ID NO:1) corresponding to amino acids 1127 to 1137 with the motif underlined.

Peptide of SEQ ID NO:1 is emulsified in complete Freund's adjuvant and injected subcutaneously at a dose of 100 µg in a series of C57BL/6 mice, followed after 2 weeks by the same injection in incomplete Freund's adjuvant. A control group is injected according to the same schedule with adjuvant alone.

Two weeks after the last injection both groups of mice are treated by a subcutaneous injection of NS5 protein in alum, repeated every 2 weeks four times.

The spleen of all mice is obtained one month after the last injection and the population of CD3+ lymphocytes isolated by FACS sorting.

Cells are then maintained in culture in the presence of antigen-presenting cells loaded with NS5 and the proportion of activated cells numbered after 6 days by incorporation of 3H-thymidine.

It is shown that the total number of NS5-specific cells is significantly higher in mice having received a CD1-restricted peptide prior to vaccination. Such higher number is observed for CD4 and, most importantly, highly significant for CD8 cells.

In parallel experiments CD3+ cells are incubated with JAWS2 as presenting cells. Such cells express high levels of surface CD1 but do not express class II MHC complexes. NS5-specific NKT cells are detected by staining with an antibody to PLZF and found only in the group of mice which has been pre-sensitized with peptide of SEQ ID NO:1.

It is therefore concluded that pre-injection of a CD1-restricted epitope in adjuvant significantly boosts the overall immune response towards NS5, but is particularly efficient in boosting a cytotoxic immune response characterized by class I-restricted CD8+ T cells and NKT cells.

As C57BL/6 and BALB/c mice present differences in terms of NKT cell frequency and distribution, the same experiment is repeated in BALB/c mice with essentially the same results.

To note, very little variation was seen in the numbers of CD4, CD8 and NKT cells when comparing individuals in groups treated by prior injection of the CD1- restricted epitope, or in between C57BL/6 and BALB/c mice, whilst large variations were observed between individual mouse in control groups of either strain.

It is concluded that pre-immunization with a CD1-restricted peptide not only increases the overall immune response towards NS5 but also reduces the differences generally observed when comparing mice of different strains.

### 2. Coxsackie B4 infection

Coxsackie B4 is a ssRNA picornavirus, which is associated with myocarditis and pancreatitis. Intraperitoneal injection of certain strains of Coxsackie, such as the B4-V, in the mouse, triggers acute pancreatitis immediately followed by a phase of chronic inflammation. In the BALB/c strain, the transition from acute to chronic inflammation is under the dependence of IL-10, a typical type 2 cytokine, and lack of IL-10 results in an abortive form of acute inflammation and absence of passage into a chronic stage.

Though Coxsackie B4-V activates innate immunity, including TLR3, TLR4 and TLR7, the virus manages to circumvent this innate response and replicates in pancreatic acini in a context of Th2 immune response. Forcing the immune response towards a cytotoxic response would prevent infection and/or at least prevent the passage from acute to chronic pancreatitis.

The amino acid sequence of Coxsackie B4 polyprotein contains a hydrophobic epitope
EK-FRDIRGF-LA (SEQ ID NO:2), corresponding to amino acids 1619 to 1629, with the CD1 binding motif underlined
BALB/c mice are immunized by footpath injection of 100 µg of peptide of SEQ ID NO:2 emulsified in Freund's adjuvant, on 2 occasions at a 2-weeks interval. A control group is injected with the adjuvant alone.

All mice are then immunized with the full polyprotein adsorbed on alum, using 100 µg per injection, for a total of 4 injections administered at an interval of 2 weeks.

Three weeks after the last injection, all mice are infected with 50 PFU of the B4-V strains in peritoneum.

Within 3 days after infection, the pancreas of 50% of the mice is isolated and examined for cellular infiltration. All mice in the control group show massive infiltration by mononuclear cells in the acini. Mice treated by injections of peptide SEQ ID NO:2 show moderate infiltration with such cells.

However, on day 10 after infection, all remaining mice in the control group show persisting cell infiltration with disappearance of acini structure, whilst mice pre-treated with peptide of SEQ ID NO:2 show complete resolution of pancreatitis signs.

It is therefore concluded that pre-immunization with peptide of SEQ ID NO:2 reduces the degree of acute acini inflammation due to B4-V infection and, more importantly, prevents the passage into a chronic irreversible stage of pancreatitis.

### 3. SARS-CoV-2 infection

SARS-CoV-2 is the causative agent of the coronavirus disease (2019 and beyond) (CoVid-19) pandemic. Observations carried out on patients' spontaneous immune response indicate a profound dysregulation of both immune response and inflammation associated with unfavorable outcome. SARS-CoV-2 has developed several strategies to circumvent innate immunity, resulting in a lack of interferon a/b production and preferential Th2 adaptive response, with pending immunopathology and risk of antibody dependent enhancement.

Reorienting the response towards an efficient innate immunity would not only provide significant protection but also prevent secondary complications.

SARS-CoV-2 cell entry depends on the interaction between virus spike protein (S protein) and angiotensin 2 receptor (ACE2). The development of an immune response to S protein would therefore constitute a suitable target for infection control.

In an attempt to mount an efficient S protein specific innate response, a peptide encompassing amino acid residues organized in a motif appropriate for presentation by CD1 is identified using algorithms as described in Patent EP3388447 A1.

Thus the sequence ATR FASVYAW NRK (SEQ ID NO:3) contains such motif (underlined), corresponding to residues 353 to 365 of the SARS-CoV-2 spike protein.

Peptide of SEQ ID NO:3 is used to immunize C57BL/6 mice at a dose of 100 µg emulsified in complete Freund's adjuvant, followed by a second injection of 100 µg in incomplete Freund's adjuvant 3 weeks later. A control group receives only the adjuvant.

Both groups of mice are then immunized with 100 µg of S protein in alum, administered at 2-week intervals, starting 3 weeks after injection of peptide of SEQ ID NO:3 (or adjuvant), for a total of 4 injections at 2 weeks interval.

The immune response towards S protein is then evaluated on splenocytes retrieved either 2 or 6 months after the last injection and titers of specific antibodies are evaluated on serum.

It is shown that mice pretreated with peptide of SEQ ID NO:3 have significantly higher numbers of CD3+ lymphocytes towards protein S than control mice. Moreover, the number of CD8+ T cells in the pretreated group is shown to outweigh the number of CD4+ T cells, whilst the reverse is seen in the control group at 2 months. Significantly, the same trend towards preferential CD8+ T cells was also observed after 6 months.

Specific anti-S protein antibody titers were evaluated at 2 and 6 months after the end of the immunization protocol, showing significantly higher titers in mice pretreated by peptide of SEQ ID NO:3 at both time points. Isotypic distribution of antibodies shows a dramatic shift from IgG1 in the control group towards IgG2a in the group pretreated with peptide of SEQ ID NO:3, and this shift was maintained after 6 months.

It is therefore concluded that pretreating mice with a peptide encompassing a CD1-compatible motif increases the immune response towards S protein, alters its characteristics towards increased cytotoxicity and Th1-driven response, and maintains this immune response over time.

### 4. Influenza virus infection

Influenza virus infection has a major impact worldwide and a significant mortality. One of the obstacles to a successful vaccination strategy is the high mutation rate of the virus, forcing to the design of a new vaccine every year. Additionally, the clinical and immunological outcomes of exposure to the virus are dictated by the past history of the individual: vaccine and/or natural exposure to the virus reactivates the response made towards previous viruses, a phenomenon called the "antigenic sin". This phenomenon renders it unlikely that a "universal" vaccine can be developed with current vaccination strategies, though some determinants of the virus are more conserved than others and could be used for such vaccination.

Creating an immune environment prone to elicitation of an innate immune response to the virus would be a significant step forward for a universal vaccine and has the property to supersede the nature and the quality of the response previously made towards vaccines and/or virus exposures.

Thus, hemagglutinin from influenza A virus contains a sequence corresponding to residues 345 to 355:
GL-FGAIAGF-IE (SEQ ID NO:4), which shows a consensus motif (underlined) able to activate NKT cells.

A synthetic peptide encompassing SEQ ID NO:4 is used at a dose of 100 µg and emulsified in Freund's adjuvant to immunize C57BL/6 mice in the footpath. This injection is repeated after 2 weeks, yet with incomplete Freund's adjuvant.

A control group of mice is injected using a similar protocol but with adjuvant only.

Three weeks after the last injection, all mice are injected with 100µg hemagglutinin adsorbed on alum and this injection is repeated 3 times at fortnight intervals.

One month after the last injection, all mice are sacrificed and lymphocyte populations are prepared from the spleen and regional lymph nodes.

Significant quantitative and qualitative differences are observed between the 2 groups of mice. The response of mice immunized with peptide of SEQ ID NO:4 is dominated by a Th1-like and cytotoxic response towards hemagglutinin, including CD8+, NK and NKT cells, as identified by lineage specific markers, whilst control mice show essentially a Th2 type immune response with little cytotoxicity participation. Accordingly, cytokine production by immunized mouse lymphocytes shows a high ratio of IFN-gamma over IL-4, whilst the reverse is observed for control mice, when the bulk lymphocyte population is incubated in the presence of histocompatible antigen-presenting cells loaded with hemagglutinin.

Isotype distribution of anti-hemagglutinin antibodies in serum shows high concentrations of IgG2a in mice immunized with peptides of SEQ ID NO:4, whilst IgG1 dominates the response made by control mice, well in accordance with the domination of a Th1-like over a Th2 response, respectively.

It is therefore concluded that pre-immunization with a peptide activating NKT cells alters the quality of the immune response obtained after administration of hemagglutinin towards a Th1-driven response and cytotoxicity.

### 5. Herpes simplex virus infection

Alpha Herpes simplex viruses (HSV) of type 1 or 2 are ubiquitous pathogens that persist for the life of infected individuals. They constitute pools of latent viruses in terminally differentiated neurons in peripheral ganglia. Reactivation in immunocompetent individuals results in self-limiting symptoms, which are paralleled to a strong Th1-like and cytotoxic immune response. However, this does not preclude severe symptoms, as well as significant morbidity, in particular in immunocompromised hosts. When the virus enters into its lytic phase, viral particles are shed and taken up by antigen-presenting cells within ganglia. The strength of the T-dependent cytotoxic activity will dictate the extent to which infection can be kept under control.

Interestingly, HSV have elaborated a strategy to prevent viral presentation by CD1d, pointing to the importance of such pathway in the defense against the virus. Further, additional mechanisms to avoid clearance by NK cells have been identified and accumulation of Th1 cells at the site of infection is directly proportional to the control of such infection. Altogether, forcing a CD1 response towards the virus, and its consequences on NK cell activation, would represent a significant step forward in the control of HSV infection.

Current vaccination using live-attenuated virus induces both B and T lymphocytes, but its overall activity in the elderly is at best of 50%, not to mention the risk of inducing a latent infection. There is therefore an important unmet medical need with respect to HSV vaccination strategies.

An epitope from the HSV Vp16 transcriptional regulatory protein contains a sequence
VL-FLHLYLF-LT (SEQ ID NO:5) corresponding to amino acid residues 179-189, with the CD1 binding motif underlined.

BALB/c mice are immunized in footpath with 100 µg of peptide of SEQ ID NO:5 emulsified in complete Freund's adjuvant, and this injection is repeated once after an interval of 2 weeks, the second injection being made in incomplete Freund's adjuvant. A control group of BALB/c mice is treated with adjuvant alone.

All mice then receive a commercial preparation of live-attenuated HSV on 2 occasions separated by one month.

One month after the last injection, the immune response towards the vaccine is compared between the 2 groups. It is shown that pre-immunization with peptide of SEQ ID NO:5 shifts the response towards a Th1-like setting, including a significant expansion of cells sharing a cytotoxicity potential, CD8+ T cells, NK cells and NKT cells. The concentration of total anti-HSV antibodies is similar in the 2 groups, but pre-immunized mice show significantly increased production of IgG2a and IgG2b specific antibodies, as demonstrated in a direct binding ELISA.

It is therefore concluded that immunization with a peptide containing a CD1-binding motif is sufficient as to shift the response towards acquisition of cytotoxicity properties helpful for the control of HSV infection.

## Claims

1. A CD1 peptide-epitope for use in the prevention or in the treatment of a disease caused by an intracellular pathogen, wherein
(i) the said disease is preferably a viral disease caused by a virus preferably selected from the group consisting of *Flaviviridae, Coronaviridae, Orthomyxoviridae, Herpesviridae and Picornaviridae,*
(ii) the said CD1 peptide-epitope is a fragment of a protein expressed by the said intracellular pathogen, wherein the said CD1 peptide-epitope comprises the sequence X₁X₂X₃X₄X₅X₆X₇, wherein the said X₂, X₃, X₄, X₅, and X₆ independently stand for any amino acid, and wherein the said X₁ and X₇ residues are independently {F;W;T;H or Y}, and
(iii) said use comprises administration of the said intracellular pathogen in an inactivated form or administration of a second peptide based on a protein of the said intracellular pathogen, wherein administration of said inactivated intracellular pathogen or said second peptide is performed subsequent to administration of the CD1 peptide-epitope and wherein said second peptide comprises the CD1 epitope sequence defined in (ii) and at least one MHC Class I epitope.

2. The CD1 peptide-epitope for use according to claim 1, wherein X₄ is {I;V;L or M} and/or wherein at least one X₁ or X₇ residue is {F;W or Y} and/or wherein the said X₁X₂X₃X₄X₅X₆X₇ sequence belongs to and/or forms or has the capacity to form an alpha-helix.

3. The CD1 peptide-epitope for use according to claim 1 or 2, wherein the CD1 peptide-epitope does not comprise an MHC class I epitope.

4. The CD1 peptide-epitope for use according to any one of the preceding claims 1 to 3, wherein the CD1 peptide-epitope does not comprise an MHC class II epitope.

5. The CD1 peptide-epitope for use according to any one of the preceding claims 1 to 4, wherein said second peptide further comprises at least one MHC Class II epitope.

6. The CD1 peptide-epitope for use according to any one of the preceding claims 1 to 5, said CD1 peptide-epitope being obtainable by an *in vitro* method for identifying one or several peptide-epitope(s) to activate NKT cells in a Th1-like and/or cytotoxic response towards an intracellular pathogen, said in vitro method comprising the steps of:
- identifying a CD1 peptide epitope in the said intracellular pathogen, and
- measuring *in vitro* the capacity of the identified CD1 peptide epitope to activate NKT cells in a Th1-like and/or cytotoxic response.

7. The CD1 peptide-epitope for use according to claim 6, wherein, in the said in vitro method, the NKT cell activation is measured upon the incubation of the peptide-epitope with cells expressing CD1 molecule at their surface, followed by the addition of a population of NKT cells and the determination of activation of said NKT cells.

8. The CD1 peptide-epitope for use according to claim 6 or 7, wherein, in the said in vitro method, the identification of the CD1 peptide epitope is measured upon the capacity of the peptide of the intracellular pathogen to bind to the said CD1 molecule.

9. The CD1 peptide-epitope for use according to any one of the preceding claims 6 to 8, wherein, in the said in vitro method, the activation of NKT cells is determined by the quantification of Interferony (IFNγ) and/or of Interleukin-12 (IL-12) levels.

10. A kit of parts for use in the treatment of a disease caused by an intracellular pathogen comprising a first peptide being the CD1 peptide epitope according to any one of the preceding claims 1 to 9 and a second peptide derived from the said intracellular pathogen and being a peptide comprising the said first peptide epitope in addition to other epitopes, preferably at least one MHC Class II epitope.

11. The kit of parts of claim 10 being for use in the treatment of a viral disease, preferably selected from the group consisting of *Flaviviridae, Coronaviridae, Orthomyxoviridae, Herpesviridae and Picornaviridae.*

12. The kit of parts of claim 10 or 11 further comprising an adjuvant for the vaccination with the first peptide and/or an adjuvant for the vaccination with the second peptide.

13. The kit of parts according to any one of the preceding claims 10 to 12, further comprising means to quantify the immunoglobulins G (IgGs, such as IgG2) specific for the CD1 peptide epitope and/or means to quantify the IgG1 and/or IgG3 specific for the CD1 peptide epitope.

14. The kit of parts according to any one of the preceding claims 10 to 13, further comprising means to quantify IL-12 IFNy,and preferably IL-4, IFNα and/or IFNβ.

15. A nucleotide sequence encoding a CD1 peptide-epitope for use in the prevention or in the treatment of a disease caused by an intracellular pathogen, wherein
(i) the said disease is preferably a viral disease caused by a virus preferably selected from the group consisting of *Flaviviridae, Coronaviridae, Orthomyxoviridae, Herpesviridae and Picornaviridae,*
(ii) the said CD1 peptide-epitope is a fragment of a protein expressed by the said intracellular pathogen, wherein the said CD1 peptide-epitope comprises the sequence X₁X₂X₃X₄X₅X₆X₇, wherein the said X₂, X₃, X₄, X₅, and X₆ independently stand for any amino acid, and wherein the said X₁ and X₇ residues are independently {F;W;T;H or Y}, and
(iii) said use comprises administration of the said intracellular pathogen in an inactivated form or administration of a second peptide based on a protein of the said intracellular pathogen, wherein administration of said inactivated intracellular pathogen or said second peptide is performed subsequent to administration of the nucleotide sequence encoding the CD1 peptide-epitope and wherein said second peptide comprises the CD1 epitope sequence defined in (ii) and at least one MHC Class I epitope.

## Patentansprüche

1. Ein CD1-Peptid-Epitop zur Verwendung bei der Vorbeugung oder Behandlung einer durch einen intrazellulären Erreger verursachten Erkrankung, wobei
(i) die Erkrankung vorzugsweise eine Viruserkrankung ist, die durch ein Virus verursacht wird, das vorzugsweise aus der Gruppe ausgewählt ist, die aus *Flaviviridae, Coronaviridae, Orthomyxoviridae, Herpesviridae* und *Picornaviridae* besteht,
(ii) das CD1-Peptid-Epitop ein Fragment eines Proteins ist, das von dem intrazellulären Pathogen exprimiert wird, wobei das CD1-Peptid-Epitop die Sequenz X₁X₂X₃X₄X₅X₆X₇ umfasst, wobei die X₂, X₃, X₄, X₅ und X₆ unabhängig voneinander für eine beliebige Aminosäure stehen und wobei X₁ und X₇ unabhängig voneinander {F; W; T; H oder Y} sind, und
(iii) die Verwendung die Verabreichung des intrazellulären Pathogens in inaktivierter Form oder die Verabreichung eines zweiten Peptids auf der Basis eines Proteins des intrazellulären Pathogens umfasst, wobei die Verabreichung des inaktivierten intrazellulären Pathogens oder des zweiten Peptids nach der Verabreichung des CD1-Peptid-Epitops erfolgt und wobei das zweite Peptid die in (ii) definierte CD1-Epitopsequenz und mindestens ein MHC-Klasse-I-Epitop umfasst.

2. Das CD1-Peptid-Epitop zur Verwendung gemäß Anspruch 1, wobei X4 {I; V; L oder M} ist und/oder wobei mindestens ein X₁- oder X₇-Rest {F; W oder Y} ist und/oder wobei die Sequenz X₁X₂X₃X₄X₅X₆X₇ zu einer Alpha-Helix gehört und/oder eine Alpha-Helix bildet oder die Fähigkeit dazu hat.

3. Das CD1-Peptid-Epitop zur Verwendung gemäß Anspruch 1 oder 2, wobei das CD1-Peptid-Epitop kein MHC-Klasse-I-Epitop umfasst.

4. Das CD1-Peptid-Epitop zur Verwendung gemäß einem der vorstehenden Ansprüche 1 bis 3, wobei das CD1-Peptid-Epitop kein MHC-Klasse-II-Epitop umfasst.

5. Das CD1-Peptid-Epitop zur Verwendung gemäß einem der vorstehenden Ansprüche 1 bis 4, wobei das zweite Peptid ferner mindestens ein MHC-Klasse-II-Epitop umfasst.

6. Das CD1-Peptid-Epitop zur Verwendung gemäß einem der vorstehenden Ansprüche 1 bis 5, wobei das CD1-Peptid-Epitop durch ein in-Vitro-Verfahren zur Identifizierung eines oder mehrerer Peptid-Epitope zur Aktivierung von NKT-Zellen in einer Th1-ähnlichen und/oder zytotoxischen Reaktion gegenüber einem intrazellulären Pathogen erhältlich ist, wobei das In-Vitro-Verfahren die folgenden Schritte umfasst:
- Identifizieren eines CD1-Peptid-Epitops in dem intrazellulären Pathogen und
- In-Vitro Messen der Fähigkeit des identifizierten CD1-Peptid-Epitops, NKT-Zellen in einer Th1-ähnlichen und/oder zytotoxischen Reaktion zu aktivieren.

7. Das CD1-Peptid-Epitop zur Verwendung gemäß Anspruch 6, wobei bei dem In-Vitro-Verfahren die NKT-Zellaktivierung nach Inkubation des Peptid-Epitops mit Zellen, die an ihrer Oberfläche das CD1-Molekül exprimieren, gemessen wird, gefolgt von der Zugabe einer Population von NKT-Zellen und der Bestimmung der Aktivierung der NKT-Zellen.

8. Das CD1-Peptid-Epitop zur Verwendung gemäß Anspruch 6 oder 7, wobei bei dem In-vitro-Verfahren die Identifizierung des CD1-Peptid-Epitops anhand der Fähigkeit des Peptids des intrazellulären Pathogens, an das CD1-Molekül zu binden, gemessen wird.

9. Das CD1-Peptid-Epitop zur Verwendung gemäß einem der vorstehenden Ansprüche 6 bis 8, wobei bei dem In-Vitro-Verfahren die Aktivierung von NKT-Zellen durch Quantifizierung der Interferon-y (IFN-γ)- und/oder Interleukin-12 (IL-12)-Spiegel bestimmt wird.

10. Ein Kit aus Teilen zur Verwendung bei der Behandlung einer durch einen intrazellulären Pathogen verursachten Erkrankung, umfassend ein erstes Peptid, das das CD1-Peptid-Epitop gemäß einem der vorstehenden Ansprüche 1 bis 9 ist, und ein zweites Peptid, das von dem intrazellulären Pathogen abgeleitet ist und ein Peptid ist, das zusätzlich zu anderen Epitopen, das erste Peptid-Epitop, vorzugsweise mindestens ein MHC-Klasse-II-Epitop, umfasst.

11. Das Kit aus Teilen gemäß Anspruch 10 zur Verwendung bei der Behandlung einer Viruserkrankung, vorzugsweise ausgewählt aus der Gruppe, die aus *Flaviviridae, Coronaviridae, Orthomyxoviridae, Herpesviridae* und *Picornaviridae* besteht.

12. Das Kit aus Teilen gemäß Anspruch 10 oder 11, der zusätzlich ein Adjuvans für die Impfung mit dem ersten Peptid und/oder ein Adjuvans für die Impfung mit dem zweiten Peptid umfasst.

13. Das Kit aus Teilen gemäß einem der vorstehenden Ansprüche 10 bis 12, ferner umfassend Mittel zur Quantifizierung der für das CD1-Peptid-Epitop spezifischen Immunglobuline G (IgGs, wie IgG2) und/oder Mittel zur Quantifizierung der für das CD1-Peptid-Epitop spezifischen IgG1 und/oder IgG3.

14. Das Kit aus Teilen gemäß einem der vorstehenden Ansprüche 10 bis 13, ferner umfassend Mittel zur Quantifizierung von IL-12, IFNγ und vorzugsweise IL-4, IFNα und/oder IFNβ.

15. Eine Nukleotidsequenz, die ein CD1-Peptid-Epitop zur Verwendung bei der Vorbeugung oder Behandlung einer durch einen intrazellulären Erreger verursachten Erkrankung codiert, wobei
(i) die Erkrankung vorzugsweise eine Viruserkrankung ist, die durch ein Virus verursacht wird, das vorzugsweise aus der Gruppe ausgewählt ist, die aus *Flaviviridae, Coronaviridae, Orthomyxoviridae, Herpesviridae* und *Picornaviridae* besteht,
(ii) das CD1-Peptid-Epitop ein Fragment eines Proteins ist, das von dem intrazellulären Pathogen exprimiert wird, wobei das CD1-Peptid-Epitop die Sequenz X₁X₂X₃X₄X₅X₆X₇ umfasst, wobei X₂, X₃, X₄, X₅ und X₆ unabhängig voneinander für eine beliebige Aminosäure stehen und wobei X₁ und X₇ unabhängig voneinander {F; W; T; H oder Y} sind, und
(iii) die Verwendung die Verabreichung des intrazellulären Pathogens in inaktivierter Form oder die Verabreichung eines zweiten Peptids auf der Basis eines Proteins des intrazellulären Pathogens umfasst, wobei die Verabreichung des inaktivierten intrazellulären Pathogens oder des zweiten Peptids nach der Verabreichung der Nukleotidsequenz, die das CD1-Peptid-Epitop codiert, erfolgt und wobei das zweite Peptid die in (ii) definierte CD1-Epitopsequenz und mindestens ein MHC-Klasse-I-Epitop umfasst.

## Revendications

1. Un peptide-épitope CD1 pour prévenir ou traiter une maladie causée par un agent pathogène intracellulaire, où
(i) ladite maladie est de préférence une maladie virale causée par un virus de préférence choisi parmi le groupe formé par les *Flaviviridae, Coronaviridae, Orthomyxoviridae, Herpesviridae* et *Picornaviridae,*
(ii) ledit peptide-épitope CD1 est un fragment d'une protéine exprimée par ledit pathogène intracellulaire, dans lequel ledit peptide-épitope CD1 comprend la séquence X₁X₂X₃X₄X₅X₆X₇, dans laquelle lesdits X₂, X₃, X₄, X₅ et X₆ représentent indépendamment n'importe quel acide aminé, et dans laquelle lesdits X₁ et X₇ sont indépendamment {F ; W ; T ; H ou Y}, et
(iii) ladite utilisation comprend l'administration dudit pathogène intracellulaire sous une forme inactivée ou l'administration d'un deuxième peptide basé sur une protéine dudit pathogène intracellulaire, dans lequel l'administration dudit pathogène intracellulaire inactivé ou dudit deuxième peptide est effectuée après l'administration du peptide-épitope CD1 et dans lequel ledit deuxième peptide comprend la séquence épitopique CD1 définie en (ii) et au moins un épitope MHC de classe I.

2. Le peptide-épitope CD1 destiné à être utilisé selon la revendication 1, dans lequel X₄ est {I ; V ; L ou M} et/ou dans lequel au moins un résidu X₁ ou X₇ est {F ; W ou Y} et/ou dans lequel ladite séquence X₁X₂X₃X₄X₅X₆X₇ appartient à et/ou forme ou a la capacité de former une hélice alpha.

3. Le peptide-épitope CD1 à utiliser selon la revendication 1 ou 2, dans lequel le peptide-épitope CD1 ne comprend pas d'épitope CMH de classe I.

4. Le peptide-épitope CD1 à utiliser selon l'une quelconque des revendications précédentes 1 à 3, dans lequel le peptide-épitope CD1 ne comprend pas d'épitope MHC de classe II.

5. Le peptide-épitope CD1 à utiliser selon l'une quelconque des revendications précédentes 1 à 4, dans lequel ledit deuxième peptide comprend en outre au moins un épitope MHC de classe II.

6. Le peptide-épitope CD1 à utiliser selon l'une quelconque des revendications 1 à 5 précédentes, ledit peptide-épitope CD1 pouvant être obtenu par une méthode *in vitro* pour identifier un ou plusieurs peptides-épitopes destinés à activer des cellules NKT dans une réponse de type Th1 et/ou cytotoxique à l'égard d'un pathogène intracellulaire, ladite méthode in vitro comprenant les étapes consistant à :
- l'identification d'un peptide-épitope CD1 dans ledit pathogène intracellulaire, et
- la mesure *in vitro* de la capacité du peptide-épitope CD1 identifié à activer des cellules NKT dans une réponse de type Thl et/ou cytotoxique.

7. Le peptide-épitope CD1 destiné à être utilisé selon la revendication 6, dans lequel, dans ladite méthode in vitro, l'activation des cellules NKT est mesurée après incubation du peptide-épitope avec des cellules exprimant la molécule CD1 à leur surface, suivie de l'ajout d'une population de cellules NKT et de la détermination de l'activation desdites cellules NKT.

8. Le peptide-épitope CD1 à utiliser selon la revendication 6 ou 7, dans lequel, dans ladite méthode in vitro, l'identification du peptide-épitope CD1 est mesurée sur la base de la capacité du peptide de l'agent pathogène intracellulaire à se lier à ladite molécule CD1.

9. Le peptide-épitope CD1 à utiliser selon l'une quelconque des revendications 6 à 8 précédentes, dans lequel, dans ladite méthode in vitro, l'activation des cellules NKT est déterminée par la quantification des niveaux d'interféron γ (IFNγ) et/ou d'interleukine-12 (IL-12).

10. Un kit de composants à utiliser dans le traitement d'une maladie causée par un pathogène intracellulaire, comprenant un premier peptide qui est le peptide-épitope CD1 selon l'une quelconque des revendications 1 à 9 précédentes et un deuxième peptide dérivé dudit pathogène intracellulaire et qui est un peptide comprenant ledit premier peptide-épitope en plus d'autres épitopes, de préférence au moins un épitope MHC de classe II.

11. Le kit de composants selon la revendication 10 destiné à être utilisé dans le traitement d'une maladie virale, de préférence choisie dans le groupe constitué par les *Flaviviridae,* les *Coronaviridae,* les *Orthomyxoviridae,* les *Herpesviridae* et les *Picornaviridae.*

12. Le kit de composants selon la revendication 10 ou 11 comprenant en outre un adjuvant pour la vaccination avec le premier peptide et/ou un adjuvant pour la vaccination avec le deuxième peptide.

13. Le kit de pièces selon l'une quelconque des revendications précédentes 10 à 12, comprenant en plus des moyens pour quantifier les immunoglobulines G (IgG, telles que IgG2) spécifiques de le peptide-épitope CD1 et/ou des moyens pour quantifier les IgG1 et/ou IgG3 spécifiques du peptide-épitope CD1.

14. Le kit de pièces selon l'une quelconque des revendications 10 à 13 précédentes, comprenant en plus des moyens pour mesurer l'IL-12, l'IFNγ et, de préférence, l'IL-4, l'IFNα et/ou l'IFNβ.

15. Une séquence nucléotidique codant pour un peptide-épitope CD1 destiné à être utilisé dans la prévention ou le traitement d'une maladie causée par un pathogène intracellulaire, dans laquelle
(i) ladite maladie est de préférence une maladie virale causée par un virus de préférence choisi dans le groupe constitué par les *Flaviviridae,* les *Coronaviridae,* les *Orthomyxoviridae,* les *Herpesviridae* et les *Picornaviridae,*
(ii) ledit peptide-épitope CD1 est un fragment d'une protéine exprimée par ledit pathogène intracellulaire, dans lequel ledit peptide-épitope CD1 comprend la séquence X₁X₂X₃X₄X₅X₆X₇, dans laquelle lesdits X₂, X₃, X₄, X₅ et X₆ représentent indépendamment n'importe quel acide aminé, et dans laquelle lesdits X₁ et X₇ sont indépendamment {F ; W ; T ; H ou Y}, et
(iii) ladite utilisation comprend l'administration dudit pathogène intracellulaire sous une forme inactivée ou l'administration d'un deuxième peptide basé sur une protéine dudit pathogène intracellulaire, dans lequel l'administration dudit pathogène intracellulaire inactivé ou dudit deuxième peptide est effectuée après l'administration de la séquence nucléotidique codant pour le peptide-épitope CD1 et dans lequel ledit deuxième peptide comprend la séquence épitopique CD1 définie en (ii) et au moins un épitope MHC de classe I.
